(19) Europäisches Patentamt
European Patent Office
Office européen des brevets

(11) **EP 3 120 926 A1**

(12) **EUROPÄISCHE PATENTANMELDUNG**

(43) Veröffentlichungstag:
**25.01.2017 Patentblatt 2017/04**

(51) Int Cl.:
*B01J 37/00* (2006.01)   *B01J 37/08* (2006.01)
*B01J 35/02* (2006.01)   *B01J 23/889* (2006.01)
*B01J 27/199* (2006.01)   *B01J 35/08* (2006.01)
*C07C 45/35* (2006.01)   *C07C 47/22* (2006.01)
*C07C 51/25* (2006.01)   *B01J 23/89* (2006.01)

(21) Anmeldenummer: **15177519.4**

(22) Anmeldetag: **20.07.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(71) Anmelder: **Evonik Degussa GmbH**
**45128 Essen (DE)**

(72) Erfinder:
• **Fischer, Achim**
  **63773 Goldbach (DE)**
• **Mescher, Axel**
  **45525 Hattingen (DE)**
• **Jakob, Harald**
  **63594 Hasselroth (DE)**

(54) **VERFAHREN ZUR HERSTELLUNG VON MISCHOXIDKATALYSATOREN**

(57) Die vorliegende Erfindung betrifft ein Verfahren zur Herstellung von Mischoxidkatalysatoren, insbesondere auf Basis von Molybdän- und Bismutoxiden, bei welchem die in Form einer Lösung und/oder Suspension vorgelegten Vorläuferverbindungen der Komponenten von Mischoxidkatalysatoren einer Sprühtrocknung mit einer spezifischen Temperaturführung unterworfen werden und die derart erhaltenen Sprühpartikel anschließend unter Erhalt einer katalytischen Aktivmasse kalziniert werden, sowie die durch dieses Verfahren erhältlichen Mischoxidkatalysatoren und die Verwendung dieser Katalysatoren in der Partialoxidation von Olefinen, insbesondere in der partiellen Gasphasenoxidation von Propan zu Acrolein und Acrylsäure. Die Sprühtrocknung der Lösung oder Suspension von Vorläuferverbindungen wird im Gleichstrom mit einem Gasstrom, der eine bestimmte Eintrittstemperatur aufweist, durchgeführt. Alternativ kann bei höherer Eintrittstemperatur eines Hauptgasstroms stromabwärts ein zusätzlicher, kälterer Gasstrom zugeführt werden. Die so hergestellten Mischoxidkatalysatoren führen beim Einsatz in der partiellen Gasphasenoxidation von Olefinen zu einer niedrigeren Maximaltemperatur im Heißpunkt des Katalysatorfestbetts.

EP 3 120 926 A1

**Beschreibung**

Die vorliegende Erfindung betrifft ein Verfahren zur Herstellung von Mischoxidkatalysatoren, die durch dieses Verfahren erhältlichen Mischoxidkatalysatoren sowie die Verwendung dieser Katalysatoren in der Partialoxidation von Olefinen.

Mischoxidkatalysatoren, insbesondere solche auf Basis von Molybdän- und Bismutoxiden, werden in der industriellen Herstellung von Acrolein und Acrylsäure bzw. Methacrolein und Methacrylsäure sowie Acrylnitril eingesetzt. Die Herstellung dieser Verbindungen erfolgt als heterogen katalysierte partielle Oxidation von Propen bzw. iso-Buten oder tert-Butanol mit Luft bzw. Ammoniak in der Gasphase an einem Katalysatorfestbett, das hauptsächlich den jeweiligen Mischoxidkatalysator enthält.

Im Rahmen der vorliegenden Erfindung werden die Begriffe Partialoxidation, partielle Gasphasenoxidation und Gasphasenpartialoxidation so verwendet, dass sie Umsetzungen organischer Verbindungen unter reaktiver Einwirkung von molekularem Sauerstoff bezeichnen, bei denen die partiell zu oxidierende organische Verbindung nach beendeter Umsetzung zumindest ein chemisch gebundenes Sauerstoffatom mehr enthält als vor der Durchführung der Partialoxidation bzw. der partiellen Gasphasenoxidation oder Gasphasenpartialoxidation.

Im Unterschied zu einer Partialoxidation, einer partiellen Gasphasenoxidation oder Gasphasenpartialoxidation wird im Rahmen der vorliegenden Erfindung der Begriff vollständige Oxidation so verwendet, dass er Umsetzungen organischer Verbindungen unter reaktiver Einwirkung von molekularem Sauerstoff bezeichnet, bei denen sämtliche in den organischen Verbindungen enthaltenen Kohlenstoffatome in Oxide des Kohlenstoffs und sämtliche in den organischen Verbindungen enthaltenden Wasserstoffatome in Oxide des Wasserstoffs umgewandelt werden.

Die zum Starten der Oxidationsreaktion erforderliche Energie wird der Reaktion über ein äußeres Wärmemedium zugeführt, im industriellen Maßstab beispielsweise über ein Salzbad, welches die von den Reaktionsgasen durchströmten den Mischoxidkatalysator enthaltenden Reaktionsrohre umgibt. Nach dem Starten der Oxidationsreaktion dient das äußere Wärmemedium in der Regel zur Abfuhr der gebildeten Reaktionswärme, da Gasphasenpartialoxidationen typischerweise exotherm verlaufen. Kommerzielle Mischoxidkatalysatoren haben üblicherweise Standzeiten von mehreren Jahren. Innerhalb dieser Zeit ist der Katalysator gealtert, das heißt, er verfügt nicht mehr über die gleiche Aktivität wie zu Beginn seines Einsatzes in der Reaktion, und dann muss das verbrauchte Katalysatorbett gegen frischen Katalysator ausgetauscht werden.

Diese Alterung wird insbesondere bei Verwendung von Mischoxidkatalysatoren, die eine Molybdän in oxidiertem Zustand enthaltende Aktivmasse aufweisen, in der Durchführung von Partialgasphasenoxidationen in Anwesenheit von Wasser bzw. Wasserdampf beobachtet. In den meisten Fällen entsteht der Wasserdampf bei der Partialgasphasenoxidation. Es wird vermutet, dass der bei der partiellen Gasphasenoxidation anwesende Wasserdampf mit dem im Katalysatorfestbett enthaltenen Molybdän in oxidiertem Zustand das leicht flüchtige $MoO(OH)_2$ bildet. In der Fachliteratur wird daher die Auffassung vertreten, dass während des Langzeitbetriebs einer heterogen katalysierten partiellen Gasphasenoxidation Molybdän kontinuierlich als $MoO_2(OH)_2$ aus der Aktivmasse des Mischoxidkatalysators heraussublimiert (vgl. Investigations of the mechanism and kinetics leading to a loss of molybdenum from bismuth molybdate catalysts, L. Zhang et al., Applied Catalysis A: General (1994), 117, 163-171). Der Verlust an der Aktivkomponente Molybdän in Mischoxidkatalysatoren ist insbesondere im Bereich der sogenannten Heißpunkte bzw. hot spots des Katalysatorfestbetts am stärksten ausgeprägt. Unter einem Heißpunkt versteht ein Fachmann der heterogenen Katalyse im Allgemeinen den Bereich eines Katalysatorfestbetts, in dem die Wärmeentwicklung der am Katalysatorfestbett in Strömungsrichtung des gasförmigen Reaktionsgemisches ablaufenden Reaktion am stärksten ist. Gegebenenfalls weist ein Katalysatorfestbett mehrere sogenannte Heißpunkte auf. Nach Durchschreiten dieser Bereiche sinkt auch die Temperatur in Strömungsrichtung der Gase deutlich, so dass sich Molybdänoxid als dünner Belag bzw. Film an den kälteren Stellen der Innenwand des Reaktionsrohres wieder niederschlägt (vgl. EP 2 155 376 A1).

Nachdem ein Bereich des Katalysatorfestbetts an der Aktivkomponente Molybdän ausgeblutet ist, wandert ein Heißpunkt entlang des Katalysatorfestbetts weiter in Bereiche, in denen die Temperatur des Katalysatorfestbetts in Strömungsrichtung der Gase zuvor niedriger war und die daher noch nicht an Molybdän ausgeblutet sind. Diese Bereiche bzw. Zonen bluten dann ebenfalls schrittweise an Molybdän aus. Aufgrund des zonenweisen Ausbluten des Katalysators an der Aktivkomponente Molybdän wird dieser Vorgang auch als Zonenalterung (im Englischen band aging) genannt.

Mit zunehmender Betriebsdauer nimmt daher nicht nur der Anteil an Molybdän in der Aktivmasse des Katalysators, sondern auch die Aktivität des Mischoxidkatalysators für die zu katalysierende Umsetzung ab. Dies macht sich in einem verminderten Umsatz des organischen Reaktanden bemerkbar. Dem Umsatzrückgang kann entgegengewirkt werden, indem die für einen spezifischen Umsatz erforderliche Temperatur des Salzbads auf einen Wert erhöht wird, der bei ansonsten unveränderten Reaktionsbedingungen denselben Umsatz für die zu oxidierende Verbindung bei einem einmaligen Durchgang durch den Reaktor erzielt wie vor der Abnahme der Aktivität des Mischoxidkatalysators.

Ein Maß für die Aktivität eines Mischoxidkatalysators bzw. eines den Mischoxidkatalysator enthaltenden Katalysatorfestbetts ist daher die Temperatur des die Reaktionsrohre mit dem Mischoxidkatalysator umgebenden Salzbads, die erforderlich ist zur Erzielung eines bestimmten Umsatzes des organischen Reaktanden, beispielsweise von Propen oder iso-Buten.

Eine Erhöhung der Temperatur des Salzbads, um der Deaktivierung eines Molybdän enthaltenden Mischoxidkatalysators entgegenzuwirken, ist jedoch kontraproduktiv für die Lebensdauer des betreffenden Mischoxidkatalysators. Denn höhere Temperaturen im Katalysatorbett beschleunigen die Alterung bzw. Deaktivierung des Katalysators, was frühzeitig entweder den vollständigen oder zumindest teilweisen Ersatz des verbrauchten Katalysatorfestbetts durch frischen Katalysator erforderlich macht. Dies führt dann zu häufigeren Wechseln des Katalysatorfestbetts und häufigeren Betriebsstillständen. Neben dem damit verbundenen Produktionsausfall stellen auch die Entsorgung des verbrauchten Katalysators sowie die Herstellung von neuem Katalysator eine erhebliche finanzielle Zusatzbelastung dar.

Zur Lösung dieser Problematik schlägt die WO 2005/049200 A1 einen aus einzelnen Sprühpulvern hergestellten ringförmigen Vollkatalysator mit einem Cobalt/Eisen-Verhältnis von 2 bis 4 und einem Cobalt/Molybdän-Verhältnis von 0,3 bis 0,7 vor. Die spezifischen Verhältnisse von Cobalt zu Eisen und von Cobalt zu Molybdän sollen in Kombination mit einander zu einer Erniedrigung der Aktivität des Katalysators führen. Dies soll zu einer Absenkung der Heißpunktemperatur führen, was sich positiv auf die Langzeitstabilität des Katalysators und die Selektivität für die Bildung von Acrolein auswirkt. Die spezifischen Verhältnisse katalytisch aktiver Elemente in der Aktivmasse schränken allerdings die Anzahl herstellbarer Katalysatoren erheblich ein.

Auch der ringförmige Mischoxidkatalysator der WO 2010/028977 A1 wird aus zwei Ausgangsmassen mit sowohl unterschiedlichen Partikeldurchmessern als auch unterschiedlichen Zusammensetzungen hergestellt. Gemäß der technischen Lehre der WO 2010/028977 A1 muss während der Herstellung des Katalysators ein bestimmter Betrag für das Produkt aus den Anteilen spezifischer Partikelgrößen der beiden Ausgangsmassen und dem stöchiometrischen Koeffizienten einer Ausgangsmasse eingehalten werden, um eine ausreichende Stabilität des Katalysators zu gewährleisten. Das damit verbundene Herstellungsverfahren ist allerdings äußerst komplex und schränkt gleichzeitig die Anzahl an herstellbaren Katalysatoren erheblich ein.

Ein weiterer Nachteil der Katalysatoren der WO 2005/049200 A1 sowie der WO 2010/028977 A1 besteht darin, dass auf Grund ihrer verringerten Aktivität für die Oxidation von Propen die Menge des nicht umgesetzten Propens ansteigt. Das nicht umgesetzte Propen muss dann als sogenanntes Kreisgas in die partielle Gasphasenoxidation zurückgeführt werden, was mit erheblichen Investitions- und Energiekosten verbunden ist. Um die Menge des Kreisgases so gering wie möglich zu halten, werden daher bei großtechnisch durchgeführten Partialgasphasenoxidationen grundsätzlich höhere Propenumsätze angestrebt als mit den Katalysatoren der WO 2005/049200 A1 bzw. der WO 2010/028977 A1 erreichbar sind.

Es besteht daher ein Bedarf an Mischoxidkatalysatoren, die eine verminderte Wärmetönung, insbesondere in den Heißpunkten eines Katalysators, während der Durchführung einer Partialgasphasenoxidation aufweisen und somit auch für den Einsatz in großtechnischen Verfahren geeignet sind.

Erfindungsgemäß wird diese Aufgabe dadurch gelöst, indem die in Form einer Lösung und/oder Suspension vorgelegten Vorläuferverbindungen der Komponenten eines Mischoxidkatalysators einer Sprühtrocknung mit einer spezifischen Temperaturführung unterworfen werden und die derart erhaltenen Sprühpartikel anschließend unter Erhalt einer katalytischen Aktivmasse kalziniert werden.

Ein Gegenstand der vorliegenden Erfindung ist daher ein Verfahren zur Herstellung eines Mischoxidkatalysators, umfassend die Schritte

a) Vorlegen einer Lösung und/oder Suspension von Vorläuferverbindungen von Komponenten des Mischoxidkatalysators,
b) Sprühtrocknen der in Schritt a) vorgelegten Lösung und/oder Suspension im Gleichstrom zusammen mit einem Gasstrom, der eine Eintrittstemperatur in den Sprühtrockner von 150 +/-10°C bis 220 +/- 10°C und eine Austrittstemperatur aus dem Sprühtrockner von 80 +/- 10°C bis 110 +/- 10°C aufweist, oder

b') Sprühtrocknen der in Schritt a) vorgelegten Lösung und/oder Suspension im Wesentlichen im Gleichstrom zusammen mit einem Hauptgasstrom, der eine Eintrittstemperatur in den Sprühtrockner von 250 +/- 10°C bis 350 +/-10°C und eine Austrittstemperatur aus dem Sprühtrockner von 110 +/- 10°C bis 140 +/- 10°C aufweist, wobei zwischen Eintritts- und Austrittspunkt des Hauptgasstroms in den Sprühtrockner bzw. aus dem Sprühtrockner ein zusätzlicher Gasstrom mit einer Eintrittstemperatur von weniger als 100°C in den Sprühtrockner zugeführt wird, und
c) Kalzinieren der aus Schritt b) oder b') erhaltenen Sprühpartikel unter Erhalt einer katalytischen Aktivmasse.

Im Rahmen der vorliegenden Erfindung wird unter einer Sprühtrocknung eine Methode zur Trocknung von Lösungen, Suspensionen oder pastösen Massen verstanden. Eine Sprühtrocknung wird in einem sogenannten Sprühturm durchgeführt, in dessen oberen Teil sich eine Sprüheinrichtung befindet. Die Sprüheinrichtung beinhaltet eine durch Flüssigkeitsdruck, Pressluft oder Inertgas betriebene Düse oder eine Zerstäuberdrehscheibe oder Drehscheibe, über die das zu trocknende Gut in einem Heißgasstrom eingebracht wird, der es in Bruchteilen einer Sekunde zu einem feinen Pulver trocknet. Damit die aus der Sprühtrocknung erhaltenen Pulver in einer anschließenden Kalzinierung in den Mischoxidkatalysator überführt werden können, muss sichergestellt werden, dass die in den Vorläuferverbindungen des Sprühpulvers enthaltenen Komponenten des herzustellenden Katalysators nicht reduziert werden. Daher wird in der erfindungsgemäßen Sprühtrocknung ein Sauerstoff enthaltendes Gas, insbesondere Luft, mit Sauerstoff angereicherte Luft oder sogar reiner Sauerstoff als Heißgas eingesetzt. Bevorzugt wird aus Kostengründen einfach Luft eingesetzt. Im erfindungsgemäßen Verfahren strömt das Heißgas entweder in Richtung mit dem Strahl, mit dem die Lösung und/oder Suspension in den Sprühturm versprüht wird, also im Gleichstrom, oder das Heißgas strömt anteilig in Richtung des Sprühstrahls und zusätzliches Heißgas strömt aus einem Punkt in der Wand des Sprühtrockners in denselben hinein. Unter einer Sprühtrocknung einer Lösung und/oder Suspension im Gleichstrom zusammen mit einem Gasstrom wird im Rahmen der vorliegenden Erfindung eine Sprühtrocknung verstanden, bei der das Heißgas in Richtung des Strahls strömt, mit dem die Lösung und/oder Suspension in den Sprühturm versprüht wird. Unter einer Sprühtrocknung einer Lösung und/oder Suspension im Wesentlichen im Gleichstrom zusammen mit einem Hauptgasstrom wird im Rahmen der vorliegenden Erfindung eine Sprühtrocknung verstanden, bei welcher der überwiegende Anteil des Heißgases in Richtung des Strahls strömt, mit dem die Lösung und/oder Suspension in den Sprühturm versprüht wird, und zusätzliches Gas in einer geringeren Menge als der Hauptgasstrom an einem Punkt in den Sprühtrockner zugeführt wird, der sich zwischen dem Eintritt- und Austrittpunkt des Hauptgasstroms in den Sprühtrockner befindet. Das anfallende Trockengut wird meist durch einen Zyklonabscheider im unteren Teil des Sprühtrockners vom Luftstrom getrennt und kann dort entnommen werden. Sprühtrockner können kontinuierlich oder diskontinuierlich betrieben werden.

Im Rahmen der vorliegenden Erfindung hat sich gezeigt, dass sowohl die Richtung in der das Heißgas durch den Sprühtrockner strömt als auch die Eintritts- und Austrittstemperatur des Heißgases einen Einfluss auf den hergestellten Mischoxidkatalysator haben. So hat beispielsweise die Richtung mit der das Heißgas durch den Sprühtrockner strömt einen Einfluss auf die Größe der Sprühpartikel. Eine Sprühtrocknung, bei der das Heißgas ausschließlich oder im Wesentlichen im Gleichstrom mit der zu trocknenden Lösung und/oder Suspension durch den Sprühtrockner strömt, generiert in der Regel kleinere Sprühpartikel als eine Sprühtrocknung, bei der das Heißgas ausschließlich oder im Wesentlichen im Gegenstrom zu der zu trocknenden Lösung und/oder Suspension durch den Sprühtrockner strömt. Es hat sich insbesondere gezeigt, dass die Sprühtrocknung mit der erfindungsgemäßen Temperaturführung Sprühpartikel generiert, die vorteilhafte Eigenschaften in die nach dem erfindungsgemäßen Verfahren hergestellten Mischoxidkatalysatoren einbringen. So führen die nach dem erfindungsgemäßen Verfahren hergestellten Mischoxidkatalysatoren beim Einsatz in der Partialgasphasenoxidation von Olefinen zu einer niedrigeren Maximaltemperatur, insbesondere im Heißpunkt des Katalysatorfestbetts, als Vergleichskatalysatoren, die in ihrer Herstellung einer von der erfindungsgemäßen abweichenden Sprühtrocknung unterzogen wurden. Auf Grund der verringerten Maximaltemperatur beim Einsatz in der Partialgasphasenoxidation von Olefinen sind die nach dem erfindungsgemäßen Verfahren hergestellten Mischoxidkatalysatoren einem geringeren Wärmestress ausgesetzt, was eine Alterung und insbesondere eine Zonenalterung des Katalysators verlangsamt.

Bei Verwendung von herkömmlichen Mischoxidkatalysatoren in der heterogen katalysierten Partialgasphasenoxidation von Olefinen wird typischerweise das Katalysatorfestbett mit Inertmaterialien verdünnt. Denn durch die Verdünnung des Katalysatorfestbetts werden sowohl die Konzentration des eingesetzten Katalysators als auch dessen Aktivität für die betreffende Partialgasphasenoxidation verringert, was gleichzeitig auch zu einer Herabsenkung der Maximaltemperatur des Katalysators in der Oxidation führt. Durch eine Verdünnung des Katalysatorfestbetts wird allerdings auch der Umsatz an Olefin sowie die Ausbeute für die gewünschte Zielverbindung - teilweise deutlich - verschlechtert. Im Gegensatz dazu erlaubt die Verwendung der nach dem erfindungsgemäßen Verfahren hergestellten Mischoxidkatalysatoren den Verzicht auf eine Verdünnung des Katalysatorfestbetts mit Inertmaterialien. Dies führt zu einer Erhöhung der Ausbeute für das gewünschte Oxidationsprodukt. Für Umsetzungen, in denen eine Verdünnung des Katalysatorfestbetts mit Inertmaterialien dennoch förderlich oder sogar unumgänglich ist, erlaubt die Verwendung der nach dem erfindungsgemäßen Verfahren hergestellten Katalysatoren einen gezielten und dosierten Einsatz von Inertmaterialien. Dadurch wird es möglich, mit kleineren als den herkömmlichen Reaktorvolumina auszukommen. Vor allen wird jedoch dadurch die Aktivität des Katalysatorfestbetts für die betreffende Partialgasphasenoxidation weniger stark als bei herkömmlichen Verfahren verringert. Der Verzicht auf Inertmaterialien oder zumindest der verringerte Einsatz von Inertmaterialien im Katalysatorfestbett erhöht daher die wirtschaftliche Attraktivität eines entsprechenden Verfahrens der Partialgasphasenoxidation.

Das erfindungsgemäße Verfahren erlaubt es daher, bereits in einem recht frühen Zeitpunkt der Katalysatorherstellung Einfluss auf die Eigenschaften des finalen Katalysators zu nehmen.

Die Vorläuferverbindungen, die in Schritt a) des erfindungsgemäßen Verfahrens mit einer Lösung und/oder Suspension vorgelegt werden, dienen als Quelle für die elementaren Bestandteile des gewünschten Mischoxids. Quellen für die elementaren Bestandteile des Mischoxids sind Verbindungen, die bereits als Oxide vorliegen und/oder durch Erhitzen, vorzugsweise in Anwesenheit von molekularem Sauerstoff und/oder Sauerstoff enthaltenden Oxidationsmitteln, in Oxide überführbar sind.

Geeignete in Oxide überführbare Verbindungen sind insbesondere solche Verbindungen, die neben den Oxiden keine weiteren Feststoffe zurücklassen, sondern anteilig zu vollständig gasförmig entweichenden Verbindungen zerfallen und/oder zersetzt werden können. Dies sind vor allem Nitrate, Formiate, Oxalate, Acetate, Carbonate, organische Aminverbindungen, Ammoniumsalze und/oder Hydroxide.

Beim Einsatz von Vorläuferverbindungen, die bereits als Oxide, beispielsweise Samariumoxid, vorliegen, wird zweckmäßigerweise eine Säure oder eine Base, bevorzugt eine Säure, hinzugegeben, um das Oxid aufzulösen oder zumindest seine Löslichkeit bzw. Suspendierbarkeit in Wasser zu verbessern. Sofern die betreffende Säure oder Base beim Erhitzen und insbesondere beim Kalzinieren nicht zu vollständig gasförmig entweichenden Verbindungen zerfällt und/oder zersetzt wird, kann durch Säure- oder Basenzugabe ein zusätzlicher Einbau von spezifischen Elementen in das Mischoxid erfolgen.

Hinsichtlich der Elemente bzw. Komponenten, die über die Vorläuferverbindungen in die gewünschten Mischoxide eingeführt werden, unterliegt das erfindungsgemäße Verfahren keinen Einschränkungen. Vielmehr werden die in Schritt a) über die entsprechenden Vorläuferverbindungen vorgelegten elementaren Bestandteile des gewünschten Mischoxids sowohl hinsichtlich ihrer Auswahl als auch hinsichtlich ihrer eingesetzten Menge über die Stöchiometrie des gewünschten Mischoxids vorgegeben. Die Stöchiometrie des Mischoxidkatalysators bzw. des im Katalysator enthaltenen Mischoxids richtet vielmehr sich nach den Verwendungen für die er gedacht ist.

Die in Partialoxidation von Olefinen eingesetzten Mischoxidkatalysatoren enthalten Molybdate unterschiedlichster Art. Unter ihnen sind üblicherweise Bismutmolybdate am stärksten vertreten. Sollen die mit dem erfindungsgemäßen Verfahren hergestellten Mischoxidkatalysatoren in der Partialoxidation von Olefinen eingesetzt werden, enthält die in Schritt a) vorgelegte Lösung bzw. Suspension zwingend eine Vorläuferverbindung von Bismut und eine Vorläuferverbindung von Molybdän oder eine Vorläuferverbindung, die sowohl Bismut als auch Molybdän enthält. Eine geeignete Quelle von Bismut ist beispielweise Bismutnitrat, und eine geeignete Quelle von Molybdän ist Ammoniumheptamolybdat. Darüber hinaus enthält eine in Schritt a) vorgelegte Lösung und/oder Suspension auch mindestens eine Vorläuferverbindung von Elementen ausgewählt aus der Gruppe bestehend aus Eisen, Wolfram, Phosphor, Kobalt, Nickel, Alkalimetall, Erdalkalimetall, Cer, Mangan, Chrom, Vanadium, Niob, Selen, Tellur, Gadolinium, Lanthan, Yttrium, Palladium, Platin, Ruthenium, Silber, Gold, Samarium, Silizium, Aluminium, Titan und Zirkonium. Vorzugsweise enthält die in Schritt a) vorgelegte Lösung und/oder Suspension Vorläuferverbindungen von den Elementen Bismut, Molybdän, Eisen, Kobalt, Nickel, Mangan, Kalium, Phosphor, Samarium und Silizium.

Hergestellt wird eine in Schritt a) vorgelegte Lösung und/oder Suspension typischerweise durch Auflösen bzw. Suspendieren der einzelnen Vorläuferverbindungen der betreffenden Elemente bzw. Komponenten in einem flüssigen Medium, das für den Einsatz in der Sprühtrocknung geeignet sein muss. Auf Grund dieses Erfordernisses wird vorzugsweise Wasser als Lösungsmittel in der Herstellung der Lösung bzw. Suspension eingesetzt. Zusätzlich können einer Lösung entsprechender Vorläuferverbindungen Säuren der eingesetzten Salze zu deren besserer Auflösung hinzugefügt werden, beispielsweise Salpetersäure, Phosphorsäure oder Kohlensäure.

Im Rahmen der vorliegenden Erfindung wird eine in Schritt a) vorgelegte Suspension vorzugsweise gemäß der folgenden Vorschrift hergestellt:

Zunächst wird eine Lösung I hergestellt, indem zunächst die Nitrate von Eisen, Kobalt, Nickel, Mangan und Kalium in Wasser gelöst werden, die erhaltene Lösung erwärmt wird, vorzugsweise auf 50°C erhitzt, und eine wässrige Lösung aus einer Sm(III)-Quelle hinzugegeben wird. Die Sm(III)-Quelle kann Samarium(III)-nitrat oder Samarium(III)-oxid sein, wobei im Fall von Samarium(III)-oxid bevorzugt Salpetersäure zur Auflösung des Oxids hinzugegeben wird.

Für eine Lösung II wird eine Molybdat-Quelle, vorzugsweise Ammoniumheptamolybdat, in erwärmtem, vorzugsweise auf 50°C aufgeheiztem, Wasser gelöst und anschließend Phosphorsäure hinzugegeben.

Ferner wird eine weitere Lösung III aus Bismutnitrat und Salpetersäure hergestellt.

Die Lösung II wird vorgelegt und zu dieser Lösung wird die Lösung III langsam und unter Rühren hinzugegeben unter Erhalt einer neuen Mischung. Durch Zugabe der Lösung I zu dieser Mischung wird ein Präzipitat erhalten, das die Mischkomponenten für die Herstellung der Aktivmasse der Katalysatoren enthält.

Die das Präzipitat enthaltende Suspension kann direkt der Sprühtrocknung zugeführt werden. Optional ist es auch möglich, das Präzipitat aus der Suspension abzutrennen, in einem anderen flüssigen Medium zu suspendieren und/oder durch Zugabe von Säuren das Präzipitat in Lösung zu bringen.

Sofern im erfindungsgemäßen Verfahren eine Suspension der Sprühtrocknung unterworfen wird, beträgt die Konzentration der Feststoffe in dieser Suspension 10 bis 50 Gew.-%, bevorzugt 20 bis 40 Gew.-%, bezogen auf die Gesamtmasse der Suspension.

Über eine Pumpe wird die Lösung und/oder Suspension aus einer Vorlage der Zerstäuberdrehscheibe zum Versprühen in den Sprühturm zugeführt. Der Durchsatz der Lösung und/oder Suspension, die auf diese Weise in den Sprühturm versprüht wird, beträgt 1 bis 6 kg/h, bevorzugt 1,5 bis 5,5 kg/h. Die Umdrehungszahl der Zerstäuberdrehscheibe liegt in einem Bereich von 30.000 U/min +/- 10% bis 50.000 U/min +/- 10%, bevorzugt bei 45.000 U/min +/- 10%.

Im Rahmen der vorliegenden Erfindung hat sich gezeigt, dass eine im Gleichstrom durchgeführte Sprühtrocknung mit einer Gaseintrittstemperatur von 160 +/- 10°C bis 200 +/- 10°C in den Sprühtrockner und einer Gasaustrittstemperatur von 90 +/- 10°C bis 105 +/- 10°C aus dem Sprühtrockner die Herstellung von Mischoxidkatalysatoren ermöglicht, die eine um annähernd 20°C niedrigere Maximaltemperatur in der Partialgasphasenoxidation von Olefinen, insbesondere von Propen, aufweisen. Ferner hat sich im Rahmen der vorliegenden Erfindung ebenfalls gezeigt, dass eine im Wesentlichen im Gleichstrom durchgeführte Sprühtrocknung mit einer Eintrittstemperatur des Hauptgasstroms von 260 +/- 10°C bis 300 +/- 10°C in den Sprühtrockner und Austrittstemperatur von 115 +/- 10°C bis 130 +/- 10°C aus dem Sprühtrockner die Herstellung von Mischoxidkatalysatoren ermöglicht, die eine um annähernd 15°C niedrigere Maximaltemperatur in der Partialgasphasenoxidation von Olefinen, insbesondere von Propen, aufweisen.

In einer Ausführungsform des erfindungsgemäßen Verfahrens weist daher in Schritt b) der Gasstrom eine Eintrittstemperatur von 160 +/- 10°C bis 200 +/- 10°C in den Sprühtrockner und eine Austrittstemperatur aus dem Sprühtrockner von 90 +/- 10°C bis 105 +/- 10°C auf. Bevorzugt weist in Schritt b) des erfindungsgemäßen Verfahrens der Gasstrom eine Eintrittstemperatur von 180 +/- 10°C bis 200 +/- 10°C in den Sprühtrockner und eine Austrittstemperatur von 90 +/- 10°C bis 105 +/- 10°C aus dem Sprühtrockner auf.

Ferner hat sich gezeigt, dass die erfindungsgemäß generierten Sprühpartikel insbesondere dann vorteilhafte Eigenschaften in die finalen Mischoxidkatalysatoren einbringen, wenn die Restfeuchte der Sprühpartikel weniger als 20 %, aber zumindest 8% beträgt. Derartige Sprühpartikel werden insbesondere bei Verwendung spezifischer Volumenströme des Heißgases zur Sprühtrocknung in Schritt b) oder b') des erfindungsgemäßen Verfahrens erhalten. Der Volumenstrom hängt mit der mittleren Strömungsgeschwindigkeit über die Beziehung

$$Q = v_a \cdot A$$

mit

$Q$: Volumenstrom in $m^3/s$,
$v_a$: mittlere Strömungsgeschwindigkeit über den Querschnitt in m/s, und
$A$: Querschnittsfläche an der Stelle in $m^2$

zusammen.

Im Rahmen des vorliegenden Erfindung werden Katalysatoren mit vorteilhaften Eigenschaften bei einer mittleren Strömungsgeschwindigkeit des Gasstromes im Sprühtrockner von 2,0 +/- 0,3 cm/s bis 4,5 +/- 0,3 cm/s in Schritt b) des erfindungsgemäßen Verfahrens und bei einer mittleren Strömungsgeschwindigkeit des Hauptgasstroms im Sprühtrockner von 2,0 +/- 0,3 cm/s bis 4,5 +/- 0,3 cm/s in Schritt b') des erfindungsgemäßen Verfahrens erhalten. Diese Strömungsgeschwindigkeiten entsprechen einem Volumenstrom von etwa 40 +/- 5 $Nm^3/h$ bis etwa 80 +/- 5 $Nm^3/h$ in Schritt b) bzw. b') des erfindungsgemäßen Verfahrens, wenn ein Sprühtrockner mit einem zylindrischen Trocknungsbereich von 0,62 m Länge und 0,8 m Durchmesser verwendet wird, beispielsweise ein Drehscheibensprühturm des Typs Mobile Minor™ der Firma GEA Niro. Die Angabe des Volumens in Normkubikmeter bezeichnet das Volumen in Kubikmetern, das das jeweilige zur Sprühtrocknung verwendete Gas bei Normbedingungen, d.h. bei 25°C und in bar, einnimmt.

In einer weiteren Ausführungsform des erfindungsgemäßen Verfahrens weist der Gasstrom in Schritt b) daher eine mittlere Strömungsgeschwindigkeit im Sprühtrockner von 2,0 +/- 0,3 cm/s bis 4,5 +/- 0,3 cm/s auf.

Vorzugsweise weist der Gasstrom in Schritt b) eine mittlere Strömungsgeschwindigkeit im Sprühtrockner von 2,5 +/- 0,3 cm/s bis 4,2 +/- 0,3 cm/s, insbesondere von 2,8 +/- 0,3 cm/s bis 4,0 +/-0,3 cm/s, auf. Diese Strömungsgeschwindigkeiten entsprechen einem Volumenstrom von etwa 45 +/-5 $Nm^3/h$ bis etwa 75 +/- 5 $Nm^3/h$ bzw. von etwa 51 +/- 5 $Nm^3/h$ bis etwa 71 +/- 5 $Nm^3/h$ bei Verwendung eines Sprühtrockners mit einem zylindrischen Trocknungsbereich von 0,62 m Länge und 0,8 m Durchmesser.

In einer alternativen Ausführungsform des erfindungsgemäßen Verfahrens weist der Hauptgasstrom in Schritt b') eine Eintrittstemperatur in den Sprühtrockner von 260 +/- 10°C bis 300 +/- 10°C und eine Austrittstemperatur aus dem Sprühtrockner von 115 +/- 10°C bis 130 +/- 10°C auf. Bevorzugt weist der Hauptgasstrom in Schritt b') eine Eintrittstemperatur in den Sprühtrockner von 270 +/- 10°C bis 290 +/- 10°C und eine Austrittstemperatur aus dem Sprühtrockner von 115 +/- 10°C bis 130 +/- 10°C auf.

In einer weiteren alternativen Ausführungsform des erfindungsgemäßen Verfahrens wird in Schritt b') ein zusätzlicher Gasstrom mit einer Eintrittstemperatur von maximal 30°C in den Sprühtrockner zugeführt.

In einer weiteren alternativen Ausführungsform des erfindungsgemäßen Verfahrens weist der Hauptgasstrom in Schritt b') eine mittlere Strömungsgeschwindigkeit im Sprühtrockner von 2,0 +/- 0,3 cm/s bis 4,5 +/- 0,3 cm/s auf. Diese Strömungsgeschwindigkeiten entsprechen einem Volumenstrom von etwa 40 +/- 5 $Nm^3/h$ bis etwa 80 +/- 5 $Nm^3/h$ bei Verwendung eines Sprühtrockners mit einem zylindrischen Trocknungsbereich von 0,62 m Länge und 0,8 m Durchmesser.

Vorzugsweise weist der Hauptgasstrom in Schritt b') eine mittlere Strömungsgeschwindigkeit im Sprühtrockner von 2,5 +/- 0,3 cm/s bis 3,5 +/- 0,3 cm/s, insbesondere von 2,8 +/- 0,3 cm/s, auf. Diese Strömungsgeschwindigkeiten entsprechen einem Volumenstrom von etwa 50 +/- 5 $Nm^3/h$ bis etwa 60 +/- 5 $Nm^3/h$ bzw. etwa 51 +/- 5 $Nm^3/h$ bei

Verwendung eines Sprühtrockners mit einem zylindrischen Trocknungsbereich von 0,62 m Länge und 0,8 m Durchmesser.

In einer zusätzlichen alternativen Ausführungsform des erfindungsgemäßen Verfahrens weist der zusätzliche Gasstrom in Schritt b') eine mittlere Strömungsgeschwindigkeit von weniger als 2,0 cm/s auf. Diese Strömungsgeschwindigkeit entspricht einem Volumenstrom von etwa 35 Nm$^3$/h bei Verwendung eines Sprühtrockners mit einem zylindrischen Trocknungsbereich von 0,62 m Länge und 0,8 m Durchmesser. Vorzugsweise weist das zusätzliche Gas in Schritt b') eine mittlere Strömungsgeschwindigkeit von maximal 1,5 +/- 0,3 cm/s und insbesondere eine Strömungsgeschwindigkeit von 1,0 +/- 0,3 cm/s auf. Diese Strömungsgeschwindigkeiten entsprechen einem Volumenstrom von etwa 27 Nm$^3$/h bzw. etwa 20 +/- 5 Nm$^3$/h bei Verwendung eines Sprühtrockners mit einem zylindrischen Trocknungsbereich von 0,62 m Länge und 0,8 m Durchmesser.

Hinsichtlich der Richtung, relativ zur Wand des Sprühtrockners, mit der der zusätzliche Gasstrom in den Sprühtrockner eintritt, unterliegt das erfindungsgemäße Verfahren prinzipiell keinen Einschränkungen. Daher kann der zusätzliche Gasstrom im Gleichstrom, im Gegenstrom, senkrecht zum Hauptgasstrom oder in Mischungen bzw. Überlagerungen dieser Richtungen in den Sprühtrockner eintreten. Gute Ergebnisse werden im Rahmen des erfindungsgemäßen Verfahrens erzielt, wenn der zusätzliche Gasstrom einen Winkel von 90 +/- 5° bis 0° relativ zur Wand des Sprühtrockners nach oben, zum oberen Ende des Sprühtrockners, in Richtung der Achse des Sprühtrockners zugeführt wird. Beträgt der Winkel des zusätzlichen Gasstromes mehr als 90°C relativ zur Wand des Sprühtrockners aus, dann zeigt der zusätzliche Gasstrom leicht nach unten, zum unteren Ende des Sprühtrockners, in Richtung der Achse des Sprühtrockners. Derartige Abweichungen von einer nach oben, zum oberen Ende des Sprühtrockners zeigenden Richtung stellen im Rahmen der vorliegenden Erfindung Fehlertoleranzen auf Grund von Verwirbelungen durch den Hauptgasstrom und ähnlichem dar. Ferner ist es im Rahmen der vorliegenden Erfindung auch möglich, den Winkel des zusätzlichen Gasstromes auch gegen die Achse des Sprühtrockners zu kippen. Wenn die mit einer Drehsprühscheibe in den Sprühtrockner versprühte Lösung und/oder Suspension eine schraubenförmige Abwärtsbewegung innerhalb des Spühtrockners beschreibt, dann ist in einer Ausführungsform der Winkel des zusätzlichen Gasstroms so von der Achse des Sprühtrockners weggekippt, dass der zusätzliche Gasstrom in die Richtung zeigt, aus welcher die in den Sprühtrockner versprühte Lösung und/oder Suspension kommt. In einer alternativen Ausführungsform ist der Winkel des zusätzlichen Gasstromes so von der Achse des Sprühtrockners weggekippt, dass der zusätzliche Gasstrom in die Richtung zeigt, in der sich die in den Sprühtrockner versprühte Lösung und/oder Suspension durch den Sprühtrocknung bewegt.

In einer Ausführungsform des erfindungsgemäßen Verfahrens wird daher der zusätzliche Gasstrom in Schritt b') in einem Winkel von 90 +/- 5° bis 0° relativ zur Wand des Sprühtrockners nach oben in Richtung der Achse des Sprühtrockners zugeführt.

Bevorzugt wird der zusätzliche Gasstrom in Schritt b') in einem Winkel von 90° bis 0°, insbesondere in einem Winkel von 90° oder 0° und besonders bevorzugt in einem Winkel von 90°, relativ zur Wand des Sprühtrockners nach oben in Richtung der Achse des Sprühtrockners zugeführt.

Auch bezüglich des Eintrittspunktes des zusätzlichen Gases in den Sprühtrockner unterliegt das erfindungsgemäße Verfahren keinen Einschränkungen. Um jedoch eine möglichst einheitliche Vermischung des Hauptgasstroms mit dem Strom des zusätzlichen Gases zu erreichen, wird der zusätzliche Gasstrom vorzugsweise an einem Punkt in den Sprühtrockner zugeführt, der sich zwischen einem Drittel oder Dreiviertel der Strecke zwischen dem Eintritts- und dem Austrittspunkt des Hauptgasstroms in den Sprühtrockner befindet. Auf diese Weise wird das gesamte für die Sprühtrocknung zur Verfügung stehende Volumen des Sprühtrockners in zwei Zonen mit einfach regulierbaren Temperaturgradienten unterteilt. Bevorzugt wird der zusätzliche Gasstrom an einem Punkt in den Sprühtrockner zugeführt, der sich auf halber Strecke zwischen dem Eintritts- und dem Austrittspunkt des Hauptgasstroms in den Sprühtrockner befindet. Dadurch wird das gesamte für die Sprühtrocknung zur Verfügung stehende Volumen des Sprühtrockners in zwei gleich große Zonen mit einfach regulierbaren Temperaturgradienten unterteilt.

In einer anderen Ausführungsform wird daher in Schritt b') der zusätzliche Gasstrom an einem Punkt in den Sprühtrockner zugeführt, der sich zwischen einem Drittel oder Dreiviertel der Strecke zwischen dem Eintritts- und dem Austrittspunkt des Hauptgasstroms in den Sprühtrockner befindet.

Die in Schritt a) des erfindungsgemäßen Verfahrens vorgelegte Lösung und/oder Suspension kann bereits Vorläuferverbindungen von sämtlichen Komponenten des Mischoxidkatalysators enthalten. Dies erlaubt die Herstellung von Mischoxidkatalysatoren direkt aus einem einheitlichen Sprühpulver. Im Gegensatz dazu werden Mischoxidkatalysatoren, insbesondere solche für die Oxidation von Olefinen, gemäß den Verfahren der WO 2005/049200 A1 und der WO 2010/028977 A1 typischerweise aus zwei Sprühpulvern mit jeweils unterschiedlicher Partikelgrößenverteilung hergestellt. Im Vergleich zu diesen Verfahren stellt das erfindungsgemäße Verfahren eine deutliche Verfahrensvereinfachung dar.

Wird die katalytische Aktivmasse für einen Mischoxidkatalysator mit nur einer einzigen Schicht katalytischer Aktivmasse chargenweise erzeugt, dann werden die Schritte des erfindungsgemäßen Verfahrens so häufig durchgeführt, wie es zur Erzeugung der erforderlichen Menge katalytischer Aktivmasse nötig ist.

In einer weiteren Ausführungsform des erfindungsgemäßen Verfahrens enthält daher die in Schritt a) vorgelegte Lösung und/oder Suspension Vorläuferverbindungen von sämtlichen Komponenten des Mischoxidkatalysators.

Alternativ ist es natürlich auch weiterhin möglich, dass die in Schritt a) vorgelegten Lösungen und/oder Suspensionen Vorläuferverbindungen von jeweils unterschiedlichen Komponenten des Mischoxidkatalysators enthalten. Dies ermöglicht beispielsweise die Herstellung von Schalenkatalysatoren mit Schichten unterschiedlicher Zusammensetzungen, beispielsweise in Form von Schichten unterschiedlicher Zusammensetzungen. Dann werden in einzelnen Sprühtrocknungen zunächst Sprühpartikel aus jeweils unterschiedlichen Lösungen hergestellt, die so erhaltenen Sprühpartikel werden einzeln für sich kalziniert und anschließend als einzelne Schichten übereinander auf einen Träger aufgebracht. Beispielsweise kann auf diese Weise ein Schalenkatalysator mit Schichten unterschiedlicher Aktivitäten für die zu katalysierende Reaktion erzeugt werden.

In einer alternativen Ausführungsform des erfindungsgemäßen Verfahrens werden in Schritt a) Lösungen und/oder Suspension mit Vorläuferverbindungen von jeweils unterschiedlichen Komponenten des Mischoxidkatalysators eingesetzt, und die Schritt a) und b) oder a) und b') werden mehrfach wiederholt.

Der nach der Kalzinierung erhaltene Mischoxidkatalysator stellt in den wenigsten Fällen den tatsächlich in der Partialgasphasenoxidation eingesetzten Katalysator dar. Vielmehr wird der aus der Kalzinierung erhaltene pulverförmige Katalysator einem Formgebungsschritt unterworfen, der den eigentlichen Katalysator liefert.

In einer weiteren Ausführungsform umfasst das erfindungsgemäße Verfahren daher zusätzlich die Schritte

d) gegebenenfalls Vermischen der aus Schritt c) erhaltenen katalytischen Aktivmassen, sofern in Schritt b) oder b') Lösungen und/oder Suspensionen von Vorläuferverbindungen jeweils unterschiedlicher Komponenten des Mischoxidkatalysators verwendet werden, unter Erhalt einer sämtliche Komponenten des Mischoxidkatalysators enthaltenden katalytischen Aktivmasse,
e) Hinzugabe eines Form- und/oder Bindemittels zu der aus Schritt c) oder d) erhaltenen katalytischen Aktivmasse,
f) Formen der aus Schritt e) erhaltenen katalytischen Aktivmasse unter Erhalt eines den Mischoxidkatalysator enthaltenden Formkörpers, und
g) Trocknen und/oder Kalzinieren und/oder Tempern des aus Schritt f) erhaltenen Katalysatorformkörpers.

Durch ein Trocknen, welches typischerweise bei Temperaturen von etwas über 100°C stattfindet, wird die gegebenenfalls in dem aus Schritt f) erhaltenen Formkörper noch enthaltene Restfeuchtigkeit entfernt. Das Kalzinieren, das typischerweise bei Temperaturen von ca. 430°C und mehr durchgeführt wird, dient zum Entfernen bzw. Abbrennen von hinzugegebenen Form- und/oder Bindemitteln aus dem Formkörper. Zudem stellt ein erneutes Kalzinieren im Herstellungsprozess sicher, dass diejenigen Komponenten des Mischoxidkatalysators, die immer noch in Form der Vorläuferverbindungen vorliegen, schließlich in die entsprechenden Oxide überführt werden. Bei einer Formgebung der pulverförmigen katalytischen Aktivmasse sollte der Katalysator vorzugsweise im Temperaturbereich von 450 bis 600°C thermisch nachbehandelt werden, damit sich die Aktivmasse für den späteren Einsatz in industriellen Reaktoren ausreichend oder überhaupt verfestigt hat. Dies erfolgt in der Regel durch ein abschließendes Tempern.

Für die industrielle Anwendung ist es besonders zweckmäßig, nach Zugabe von handelsüblichen Form- und Bindemitteln, die pulverförmigen katalytische Aktivmasse in Form zu bringen. Dies kann durch Tablettierung, Extrusion oder durch Beschichtung eines Trägers geschehen. Der Träger ist hierbei hinsichtlich seiner geometrischen Form nicht auf spezifischen Formen bzw. auf eine bestimmte Anzahl von Formen limitiert. Vielmehr richtet sich die geometrische Form des Trägers nach den Vorgaben des Reaktors (z.B. Rohrdurchmesser, Länge der Katalysatorschüttung etc.). Beispielsweise kann der Träger die Form eines Zylinders, einer Kugel, einer Pyramide, eines Sattels oder eines Polyeders haben, er kann aber auch eine Wand eines Reaktionsraumes darstellen. Vorzugsweise hat der Träger die Form einer Kugel.

Als Bindemittel können diverse Öle, Cellulose-Derivate, Polyvinylalkohole, Saccharide, Acrylate sowie deren Alkyl-Derivate, Mischungen oder Kondensate aus denselben eingesetzt werden. Bevorzugt werden Acrylate, Polyvinylkohole sowie Cellulose-Derivate eingesetzt und besonders bevorzugt Derivate und Kondensate von Acrylaten und/oder Cellulosen sowie Mischungen derselben.

Alternativ ist es auch möglich, aus der aus Schritt c) erhaltenen katalytischen Aktivmasse eine sogenannte Washcoat-Suspension herzustellen, diese auf ein Trägermaterial aufzubringen und durch Trocknen und/oder Kalzinieren das beschichtete Trägermaterial in einen geträgerten Mischoxidkatalysator zu überführen. Im Rahmen der vorliegenden Erfindung bezeichnet daher eine Washcoat-Suspension jede Art von Suspension, die eine nach dem erfindungsgemäßen Verfahren hergestellte oder erhältliche katalytische Aktivmasse enthält.

In einer alternativen Ausführungsform umfasst das erfindungsgemäße Verfahren daher zusätzlich die Schritte

h) Bereitstellen einer Washcoat-Suspension enthaltend die aus Schritt c) erhaltene katalytische Aktivmasse,
i) Aufbringen der Washcoat-Suspension aus Schritt h) auf ein Trägermaterial, und
j) Trocknen und/oder Kalzinieren und/oder Tempern des aus Schritt i) erhaltenen beschichteten Trägermaterials unter Erhalt eines geträgerten Mischoxidkatalysators.

Vorzugsweise werden der Washcoat-Suspension in Schritt h) handelsübliche - vorstehend beschriebene - Form- und Bindemitteln hinzugegeben, um eine Fixierung der pulverförmigen katalytischen Aktivmasse auf dem Trägermaterial zu erleichtern bzw. zu ermöglichen.

Auch in dieser alternativen Ausführungsform des erfindungsgemäßen Verfahrens ist der Träger hinsichtlich seiner geometrischen Form nicht auf spezifischen Formen bzw. auf eine bestimmte Anzahl von Formen limitiert. Vielmehr richtet sich die geometrische Form des Trägers nach den Vorgaben des Reaktors (z.B. Rohrdurchmesser, Länge der Katalysatorschüttung etc.). Beispielsweise kann der Träger die Form eines Zylinders, einer Kugel, einer Pyramide, eines Sattels oder eines Polyeders haben, er kann aber auch eine Wand eines Reaktionsraumes darstellen. Vorzugsweise hat der Träger die Form einer Kugel.

Ein weiterer Gegenstand der vorliegenden Erfindung ist zudem auch eine Washcoat-Suspension, die eine nach dem erfindungsgemäßen Verfahren erhaltene oder erhältliche katalytische Aktivmasse enthält.

Zur Herstellung der erfindungsgemäßen Washcoat-Suspension wird eine nach dem erfindungsgemäßen Verfahren hergestellte oder erhältliche katalytische Aktivmasse in entionisiertem Wasser aufgeschlämmt. Falls erforderlich, wird die so erhaltene Washcoat-Suspension einer Behandlung mit einem Dispergiergerät unterzogen, um eine gleichmäßige Partikelverteilung in der Suspension zu gewährleisten. Anschließend wird ein Bindemittel, wie vorstehend behandelt, zur Washcoat-Suspension hinzugegeben.

Im einfachsten Fall erfolgt das Aufbringen einer erfindungsgemäßen Washcoat-Suspension auf ein Trägermaterial durch Aufsprühen der Washcoat-Suspension. Vorzugsweise wird das Aufbringen der Washcoat-Suspension so durchgeführt, dass auf eine einer Umwälzbewegung unterzogenen Schüttung von Trägerkörpern die in Schritt h) bereitgestellte bzw. die erfindungsgemäße Washcoat-Suspension aufgesprüht wird.

Der auf diese Weise erhaltene geträgerte Mischoxidkatalysator enthält einen Trägerkörper und eine diesen Trägerkörper einhüllende Schale mit katalytisch aktivem Material. Derartige nach dem erfindungsgemäßen Verfahren erhaltene geträgerte Trägerkatalysator werden auch als Schalenkatalysator bezeichnet.

Die nach dem erfindungsgemäßen Verfahren erhaltenen oder erhältlichen Mischoxidkatalysatoren eignen sich für die katalysierte Partialgasphasenoxidation von Olefinen zu ungesättigten Aldehyden und/oder ungesättigten Säuren.

Vorzugsweise entspricht die nach dem erfindungsgemäßen Verfahren erhaltene oder erhältliche katalytische Aktivmasse der allgemeinen Formel

$$(Mo_{12}Bi_aFe_b(Ni+Co)_cD_dE_eF_fG_gH_h)O_x \qquad (I),$$

wobei

Mo     Molybdän bedeutet,

Bi      Bismut bedeutet,

D      Wolfram und/oder Phosphor ist,

E      mindestens eine der Komponenten ausgewählt aus der Gruppe bestehend aus Lithium, Kalium, Natrium, Rubidium, Cäsium, Magnesium, Calcium, Barium und Strontium,

F      mindestens eine der Komponenten ausgewählt aus der Gruppe bestehend aus Cer, Mangan, Chrom und Vanadium,

G      mindestens eine der Komponenten ausgewählt aus der Gruppe bestehend aus Niob, Selen, Tellur, Samarium, Gadolinium, Lanthan, Yttrium, Palladium, Platin, Ruthenium, Silber und Gold,

H      mindestens einer der Komponenten ausgewählt aus der Gruppe bestehend aus Silizium, Aluminium, Titan und Zirkonium,

O      Sauerstoff bedeutet,

und

a =     0 bis 5,0

b =     0,5 bis 5,0

c =     2 bis 15

d =     0.01 bis 5,0

e =     0,001 bis 2

f =     0,001 bis 5

g =     0 bis 1,5

h =     0 bis 800,

und

x =     eine Zahl, die von der Wertigkeit und der Häufigkeit der von Sauerstoff verschiedenen Komponenten bestimmt wird.

Besonders bevorzugt entspricht die nach dem erfindungsgemäßen Verfahren erhaltene oder erhältliche katalytische Aktivmasse der Zusammensetzung $(Mo_{12}Bi_{1,5}(Co+Ni)_{8,0}Fe_{1,8}Mn_{0,01}K_{0,06}P_{0,04}Si_{0,66}Sm_{0,1})O_x$.

Ein weiterer Gegenstand der vorliegenden Erfindung ist daher auch eine nach dem erfindungsgemäßen Verfahren erhaltene oder erhältliche katalytische Aktivmasse.

Ein weiterer Gegenstand der vorliegenden Erfindung ist daher auch die Verwendung eines nach dem erfindungsgemäßen Verfahren hergestellten oder erhältlichen Mischoxidkatalysators in der Herstellung von ungesättigten Aldehyden und/oder Säuren durch Partialoxidation von Olefinen bzw. ein Verfahren zur Herstellung von ungesättigten Aldehyden und/oder Säuren durch Partialoxidation von Olefinen, bei welchem die Olefine in Anwesenheit eines nach dem erfindungsgemäßen Verfahren hergestellten oder erhältlichen Mischoxidkatalysators oder eines erfindungsgemäßen Mischoxidkatalysators oxidiert werden.

Die Partialoxidation von Olefinen wird im Allgemeinen bei Temperaturen von 250 bis 450°C und einem Druck von 1 bis 3 bara durchgeführt. Dabei werden die Reaktionspartner Olefin, Luft bzw. Sauerstoff oder mit Sauerstoff angereicherte Luft und Inertgase bevorzugt im molaren Verhältnis 1:6-9:3-18 bei einer Belastung von 2 bis 10 mol Olefin pro Liter Katalysatorschüttung pro Stunde der Katalysatorschüttung zugeführt.

Vorzugsweise ist das Olefin ein $C_1$-$C_4$ Olefin, besonders bevorzugt handelt es sich bei dem Olefin um Propen. Propen wird zur Herstellung von Acrolein und Acrylsäure vor allem als chemical grade oder polymer grade eingesetzt, es kann aber auch refinery grade Propen benutzt werden. Als Inertgase lassen sich alle gasförmigen Verbindungen einsetzen, die sich unter den beschriebenen Reaktionsbedingungen inert verhalten. Beispielsweise können die Inertgase Stickstoff, Helium, Ethan, Propan, oder Gemische davon sein. Ebenfalls möglich ist eine Kreisführung von nicht in der Reaktion umgesetzten Komponenten, die durch Auskondensieren aus dem Produktgasgemisch abgetrennt werden.

Besonders gute Ergebnisse werden beim Einsatz von Rohrbündel-, Platten- oder Wandreaktoren erhalten, bei letzterem ist der Katalysator auf die Wand aufgebracht.

**Beispiele:**

**I. Herstellung von Mischoxidkatalysatoren**

**1.1 Bereitstellung von Suspensionen für die Katalysatorherstellung**

**Beispiel 1:**

Eine erste Lösung (im Folgenden als Lösung I bezeichnet) wurde hergestellt, indem zunächst die Nitrate von Eisen, Kobalt, Nickel, Mangan und Kalium in den Massenanteilen 23,2 : 47,26, : 29,28 : 0,0646 : 0,2067 in 3,5 Litern Wasser gelöst wurden, die erhaltene Mischung unter Rühren auf 50°C erhitzt und anschließend eine salpetersaure Lösung von 0,1 $Sm^{3+}$ und 2 mol $HNO_3$ hinzugegeben wurde.

Eine separate zweite Lösung (im Folgenden als Lösung II bezeichnet) wurde hergestellt durch Auflösen von 2.118,6 g Ammoniumheptamolybdat in 2,7 Litern Wasser und anschließende Zugabe einer Lösung von 4,4 g Phosphorsäure in 1 Liter Wasser.

Eine weitere separate Lösung (im Folgenden als Lösung III bezeichnet) wurde hergestellt aus 1.280 g Bismutnitrat und 0,72 mol $HNO_3$.

Die Lösung II wurde vorgelegt, und zu dieser Lösung wurde langsam, unter Rühren die Lösung III hinzugegen. Zu der derart erhaltenen Mischung wurde die Lösung I unter Erhalt einer Suspension hinzugegeben.

**1.2 Herstellung von Aktivmassen für die Katalysatoren**

Sämtliche Aktivmassen für die Katalysatoren wurden durch Sprühtrocknung in einem Drehscheibenturm des Typs Mobile Minor™ (http://www.niro.com/niro/cmsdoc.nsf/WebDoc/ndkk5j9c7h) der Firma GEA-Niro (GEA Germany, GEA Group Aktiengesellschaft, Peter-Müller-Str. 12, 40468 Düsseldorf, Germany) hergestellt. Dieser Sprühtrockner weist einen zylindrischen Abschnitt mit einem Durchmesser von 0,8 m und einer Höhe von 0,62 m auf, in welchem die Sprühtrocknung erfolgt, und einen sich an dessen unterem Ende anschließenden umgedrehten kegelförmigen Abschnitt mit einer Länge von 0,72 m auf, der zum Sammeln der generierten Sprühpartikel dient.

Die Bestimmung der spezifischen Partikelgrößenverteilung erfolgt erfindungsgemäß in Nass-Dispergierung mittels Laserstreuung gemäß dem internationalen Standard ISO 13320. Im Rahmen der vorliegenden Erfindung erfolgte die Bestimmung der Partikelgrößenverteilung mit einem Particle Size Analyzer der Serie LS™ 13320 und einem Universal Liquid Module, beides von Beckmann Coulter (Brea, Kalifornien) unter Verwendung von PIDS (Polarization Intensity Differential Scattering)-Daten. Die Sprühpartikel wurden in Ethanol dispergiert und ohne Ultraschallbadbehandlung mit einer relativen Pumpgeschwindigkeit von ca. 31% der Bestimmung der Partikelgrößenverteilung zugeführt. Das optische Modell zur Partikelgrößenbestimmung ist das Fraunhofer-Modell. Die Messzeit für die Partikelgrößenbestimmung beträgt ca. 82 Sekunden.

Die als Messergebnis angegebenen Partikeldurchmesser $d_x$ sind so definiert, dass X % des Gesamtpartikelvolumens aus Partikeln mit einem kleinerem Durchmesser bestehen. Das heißt, (100-X) % des Gesamtpartikelvolumens bestehen aus Partikeln mit einem Durchmesser $\geq d_x$.

**Beispiel 2a (erfindungsgemäß):**

Die in Beispiel 1 erhaltene Suspension wurde in einem Drehscheibensprühtrockner des Typs Mobile Minor™ der Firma GEA-Niro getrocknet: Die Suspension wurde mit einer Dosierate von 2 +/- 0,1 l/h zusammen mit 51 +/- 2,5 $Nm^3$/h Luft (entspricht einer mittleren Strömungsgeschwindigkeit im Sprühtrockner von etwa 2,8 +/- 0,3 cm/s), die eine Eintrittstemperatur in den Sprühtrockner von 200°C aufwies, über eine Drehscheibe mit einer Umdrehungszahl von 45.000 $min^{-1}$ von oben nach unten im Gleichstrom mit der eintretenden Luft in den Sprühtrockner versprüht. Die Austrittstemperatur der Luft aus dem Sprühtrockner betrug 105°C. Die erhaltenen, sprühgetrockneten Partikel wiesen eine Restfeuchte von 14,5% auf. Die Partikelgrößenverteilung der erhaltenen Sprühpulver betrug $d_5$ = 11,22 $\mu$m, $d_{10}$ = 13,86 $\mu$m, $d_{50}$ = 26,33 $\mu$m, $d_{90}$ = 46,39 $\mu$m und $d_{95}$ = 56,90 $\mu$m. Der Mittelwert der Partikelgrößenverteilung betrug 29,39 $\mu$m und der Medianwert 26,33 $\mu$m. Das Maximum der Partikelgrößenverteilung lag bei 28,70 $\mu$m.

**Beispiel 2b (erfindungsgemäß):**

Die in Beispiel 1 erhaltene Suspension wurde in einem Drehscheibensprühtrockner des Typs Mobile Minor™ der Firma GEA-Niro getrocknet: Die Suspension wurde mit einer Dosierate von 2 +/- 0,1 l/h zusammen mit 51 +/- 2,5 $Nm^3$/h Luft (entspricht einer mittleren Strömungsgeschwindigkeit im Sprühtrockner von etwa 2,8 +/- 0,3 cm/s), die eine Eintrittstemperatur in den Sprühtrockner von 180°C aufwies, über eine Drehscheibe mit einer Umdrehungszahl von 45.000 $min^{-1}$ von oben nach unten im Gleichstrom mit der eintretenden Luft in den Sprühtrockner versprüht. Die Austrittstemperatur der Luft aus dem Sprühtrockner betrug 92 +/- 5°C. Die erhaltenen, sprühgetrockneten Partikel wiesen eine Restfeuchte von 16,4% auf. Die Partikelgrößenverteilung der erhaltenen Sprühpulver betrug $d_5$ = 10,74 $\mu$m, $d_{10}$ = 16,20 $\mu$m, $d_{50}$ = 33,00 $\mu$m, $d_{90}$ = 60,74 $\mu$m und $d_{95}$ = 91,39 $\mu$m. Der Mittelwert der Partikelgrößenverteilung betrug 37,99 $\mu$m und der Medianwert 33,00 $\mu$m. Das Maximum der Partikelgrößenverteilung lag bei 34,59 $\mu$m.

**Beispiel 2c (erfindungsgemäß):**

Die in Beispiel 1 erhaltene Suspension wurde in einem Drehscheibensprühtrockner des Typs Mobile Minor™ der Firma GEA-Niro getrocknet: Die Suspension wurde mit einer Dosierate von 2 +/- 0,1 l/h zusammen mit 71 +/- 2,5 $Nm^3$/h Luft (entspricht einer mittleren Strömungsgescheindigkeit im Sprühtrockner von etwa 4,0 +/- 0,3 cm/s), die eine Eintrittstemperatur in den Sprühtrockner von 180°C aufwies, über eine Drehscheibe mit einer Umdrehungszahl von 45.000 $min^{-1}$ von oben nach unten im Gleichstrom mit der eintretenden Luft in den Sprühtrockner versprüht. Die Austrittstemperatur der Luft aus dem Sprühtrockner betrug 99 +/- 5°C. Die erhaltenen, sprühgetrockneten Partikel wiesen eine Restfeuchte von 14% auf. Die Partikelgrößenverteilung der erhaltenen Sprühpulver betrug $d_5$ = 13,20 $\mu$m, $d_{10}$ = 16,16 $\mu$m, $d_{50}$ = 30,25 $\mu$m, $d_{90}$ = 51,33 $\mu$m und $d_{95}$ = 63,76 $\mu$m. Der Mittelwert der Partikelgrößenverteilung betrug 33,71 $\mu$m und der Medianwert 30,25 $\mu$m. Das Maximum der Partikelgrößenverteilung lag bei 31,51 $\mu$m.

**Beispiel 3a (Vergleichsbeispiel):**

Die Suspension von Beispiel 1 wurde in einem Drehscheibensprühtrockner des Typs Mobile Minor™ der Firma GEA-Niro getrocknet: Die Suspension wurde mit einer Dosierate von 2 +/- 0,1 l/h zusammen mit 51 +/- 2,5 $Nm^3$/h Luft (entspricht einer mittleren Strömungsgeschwindigkeit im Sprühtrockner von etwa 2,8 +/- 0,3 cm/s), die eine Eintrittstemperatur in den Sprühtrockner von 300°C aufwies, über eine Drehscheibe mit einer Umdrehungszahl von 45.000 $min^{-1}$ von oben nach unten im Gleichstrom mit der eintretenden Luft in den Sprühtrockner versprüht. Die Austrittstemperatur der Luft aus dem Sprühtrockner betrug 180 +/- 5°C. Die erhaltenen, sprühgetrockneten Partikel wiesen eine Restfeuchte von 2,2% auf. Die Partikelgrößenverteilung der erhaltenen Sprühpulver betrug $d_5$ = 0,503 $\mu$m, $d_{10}$ = 6,970 $\mu$m, $d_{50}$ = 19,87 $\mu$m, $d_{90}$ = 37,10 $\mu$m und $d_{95}$ = 41,86 $\mu$m. Der Mittelwert betrug 21,31 $\mu$m und der Medianwert 19,87 $\mu$m. Das Maximum der Partikelgrößenverteilung lag bei 19,76 $\mu$m.

**Beispiel 3b (Vergleichsbeispiel):**

Die Suspension von Beispiel 1 wurde in einem Drehscheibensprühtrockner des Typs Mobile Minor™ der Firma GEA-Niro getrocknet: Die Suspension wurde mit einer Dosierrate von 2 +/- 0,1 l/h zusammen mit 51 +/- 2,5 Nm$^3$/h Luft (entspricht einer mittleren Strömungsgeschwindigkeit im Sprühtrockner von etwa 2,8 +/- 0,3 cm/s), die eine Eintrittstemperatur in den Sprühtrockner von 212°C aufwies, über eine Drehscheibe mit einer Umdrehungszahl von 45.000 min$^{-1}$ von oben nach unten im Gleichstrom mit der eintretenden Luft in den Sprühtrockner versprüht. Die Austrittstemperatur der Luft aus dem Sprühtrockner betrug 120°C. Die erhaltenen, sprühgetrockneten Partikel wiesen eine Restfeuchte von 6,4% auf. Die Partikelgrößenverteilung der erhaltenen Sprühpulver betrug $d_5$ = 3,25 $\mu$m, $d_{10}$ = 10,54 $\mu$m, $d_{50}$ = 24,90 $\mu$m, $d_{90}$ = 45,86 $\mu$m und $d_{95}$ = 59,07 $\mu$m. Der Mittelwert betrug 27,81 $\mu$m und der Medianwert 24,90 $\mu$m. Das Maximum der Partikelgrößenverteilung lag bei 28,70 $\mu$m.

**Beispiel 4a (erfindungsgemäß):**

Die Suspension aus Beispiel 1 wurde in einem Drehscheibensprühtrockner des Typs Mobile Minor™ der Firma GEA-Niro getrocknet: Die Suspension wurde mit einer Dosierrate von 2 +/- 0,1 l/h zusammen mit 51 +/- 2,5 Nm$^3$/h Luft (entspricht einer mittleren Strömungsgeschwindigkeit im Sprühtrockner von etwa 2,8 +/- 0,3 cm/s), die eine Eintrittstemperatur in den Sprühtrockner von 275°C aufwies, über eine Drehscheibe mit einer Umdrehungszahl von 45.000 min$^{-1}$ von oben nach unten im Gleichstrom mit der eintretenden Luft in den Sprühtrockner versprüht. Über eine senkrechte Zuleitung auf halber Höhe des Trockenraums des Sprühtrockners wurden 20 Nm$^3$/h zusätzliche Luft (entspricht einer mittleren Strömungsgeschwindigkeit im Sprühtrockner von etwa 1,0 +/- 0,3 cm/s), die eine Eintrittstemperatur in den Sprühtrockner von 20°C aufwies, in einem Winkel von 90° zur Trocknerwand in den Sprühtrockner versprüht. Die Austrittstemperatur der Luft aus dem Sprühtrockner betrug 125°C, unabhängig davon ob sie von oben nach unten oder von der Seite in den Sprühtrockner eintrat. Die erhaltenen, sprühgetrockneten Partikel wiesen eine Restfeuchte von 11,2% auf. Die Partikelgrößenverteilung der erhaltenen Sprühpulver betrug $d_5$ = 9,646 $\mu$m, $d_{10}$ = 12,30 $\mu$m, $d_{50}$ = 23,00 $\mu$m, $d_{90}$ = 38,93 $\mu$m und $d_{95}$ = 44,58 $\mu$m. Der Mittelwert betrug 24,94 $\mu$m und der Medianwert 23,00 $\mu$m. Das Maximum der Partikelgrößenverteilung lag bei 23,82 $\mu$m.

**Beispiel 4b (erfindungsgemäß):**

Die Suspension aus Beispiel 1 wurde in einem Drehscheibensprühtrockner des Typs Mobile Minor™ der Firma GEA-Niro getrocknet: Die Suspension wurde mit einer Dosierrate von 2 +/- 0,1 l/h zusammen mit 51 +/- 2,5 Nm$^3$/h Luft (entspricht einer mittleren Strömungsgeschwindigkeit im Sprühtrockner von etwa 2,8 +/- 0,3 cm/s), die eine Eintrittstemperatur in den Sprühtrockner von 275°C aufwies, über eine Drehscheibe mit einer Umdrehungszahl von 45.000 min$^{-1}$ von oben nach unten im Gleichstrom mit der eintretenden Luft in den Sprühtrockner versprüht. Über eine senkrechte Zuleitung auf halber Höhe des Trockenraums des Sprühtrockners wurden 20 Nm$^3$/h (entspricht einer mittleren Strömungsgeschwindigkeit im Sprühtrockner von etwa 1,0 +/- 0,3 cm/s) zusätzliche Luft, die eine Eintrittstemperatur in den Sprühtrockner von 20°C aufwies, von oben nach unten (in einem Winkel von 0° zur Trocknerwand des Sprühtrockners in Richtung des Hauptluftstroms) in den Sprühtrockner versprüht. Die Austrittstemperatur der Luft aus dem Sprühtrockner betrug 125°C, unabhängig davon ob sie von oben nach unten oder von der Seite in den Sprühtrockner eintrat. Die erhaltenen, sprühgetrockneten Partikel wiesen eine Restfeuchte von 11,2% auf. Die Partikelgrößenverteilung der erhaltenen Sprühpulver betrug $d_5$ = 2,046 $\mu$m, $d_{10}$ = 8,712 $\mu$m, $d_{50}$ = 22,00 $\mu$m, $d_{90}$ = 38,66 $\mu$m und $d_{95}$ = 44,54 $\mu$m. Der Mittelwert betrug 23,60 $\mu$m und der Medianwert 22,00 $\mu$m. Das Maximum der Partikelgrößenverteilung lag bei 38,82 $\mu$m.

**1.3 Herstellung von Katalysatoren aus sprühgetrockneten Pulvern**

**Beispiel 5:**

Die in den Beispielen 2a bis 2c, 3a bis 3b und 4a bis 4b hergestellten Pulver wurden in einem Umluftofen bei einer Temperatur von 430 +/- 5°C für die Dauer von einer Stunde kalziniert. Die erhaltenen Mischoxide wurden dann als wässrige Suspension auf einen keramischen, kugelförmigen Katalysatorträger aufgesprüht und bei 60°C im Luftstrom getrocknet. Die so erhaltenen Pellets wurden anschließend zur Homogenisierung in einer Trommel umgewälzt. Zur Verfestigung der aufgebrachten Aktivmasse wurde das erhaltene Gut noch für die Dauer von 5 Stunden einer Wärmebehandlung bei einer Temperatur von 520°C unterworfen. Alle auf diese Weise hergestellten Katalysatoraktivmassen wiesen die Zusammensetzung $(Mo_{12}Bi_{1,5}(Co+Ni)_{8,0}Fe_{1,8}Mn_{0,01}K_{0,06}P_{0,04}Si_{0,66}Sm_{0,1})O_x$ auf.

**II. Testung der Mischoxidkatalysatoren**

**Beispiel 6:**

Die in Beispiel 5 aus den Aktivmassen der Beispiele 2a bis 2c und 3a bis 3b hergestellten Katalysatoren wurden in der partiellen Gasphasenoxidation von Propen eingesetzt. Hierfür wurde ein Rohrreaktor mit einem Rohrrinnendurchmesser von 20,5 mm mit einem Katalysatorfestbett mit einer Länge von jeweils 285 cm beschickt. Der Rohrreaktor wurde von einem Bad umgeben, mit dem die Temperatur des Katalysatorfestbetts bzw. des das Katalysatorfestbett durchströmenden Reaktionsgemisches reguliert wurde. Ein Feedgasstrom aus Propen (ca. 7 Vol.-%, chemical grade), Luft und Inertstoffen wurde in den Rohrreaktor eingeleitet und das Propen an dem Katalysatorfestbett umgesetzt. Die zugeführte Menge an Propen wurde so gewählt, dass der Quotient aus Propengasmenge in Litern pro Stunde und der eingesetzten Füllmenge des Katalysators in Litern einen Wert von 146 $h^{-1}$ hat. Der Propenumsatz wurde durch die Wahl der Badtemperatur auf einen Wert von 97 +/- 0,5% eingestellt. Ferner wurde die Luftmenge zur Oxidation so eingestellt, dass bei dem eingestellten Propenumsatz der Restsauerstoffanteil im Gasstrom nach einmaligem Durchgang durch den Reaktor 6 Vol.-% betrug. Bei allen Katalysatoren betrug die Summenausbeute für Acrolein und Acrylsäure immer mehr als 90%, wobei die Ausbeute für Acrolein immer mindestens 84% betrug. Bei allen Tests mit den Katalysatoren auf Basis der Aktivmassen der Beispiele 2a bis 2c und 3a bis 3b wurde die jeweilige maximale Temperatur im Katalysatorfestbett ermittelt.

Tabelle 1: Übersicht über die Temperaturen in den Reaktionen des Beispiels 6.

| Katalysator (aus Beispiel) | GHSV [$h^1$] | Propylen [Vol.-%] | $T_{Bad,Reaktor}$ [°C] | $T_{max,Reaktor}$ [°C] |
|---|---|---|---|---|
| 2a (erfindungsgemäß) | 2057 | 7,09 | 348 | 422 |
| 2b (erfindungsgemäß) | 2051 | 7,12 | 347 | 422 |
| 2c (erfindungsgemäß) | 2046 | 7,15 | 355 | 419 |
| 3a (Vergleichsbeispiel) | 2036 | 7,16 | 352 | 437 |
| 3b (Vergleichsbeispiel) | 2025 | 7,21 | 360 | 435 |

**Beispiel 7:**

Die in Beispiel 5 aus den Aktivmassen der Beispiele 2a bis 2c und 3a bis 3b hergestellten Katalysatoren wurden in der partiellen Gasphasenoxidation von Propen eingesetzt. Hierfür wurde ein Rohrreaktor mit einem Rohrrinnendurchmesser von 20,5 mm mit einem Katalysatorfestbett mit einer Länge von jeweils 285 cm beschickt. Der Rohrreaktor wurde von einem Bad umgeben, mit dem die Temperatur des Katalysatorfestbetts bzw. des das Katalysatorfestbett durchströmenden Reaktionsgemisches reguliert wurde. Ein Feedgasstrom aus Propen (ca. 7 Vol.-%, chemical grade), Luft und Inertstoffen wurde in den Rohrreaktor eingeleitet und das Propen an dem Katalysatorfestbett umgesetzt. Die zugeführte Menge an Propen wurde so gewählt, dass der Quotient aus Propengasmenge in Litern pro Stunde und der eingesetzten Füllmenge des Katalysators in Litern einen Wert von 153 $h^{-1}$ hat. Der Propenumsatz wurde durch die Wahl der Badtemperatur auf einen Wert von 97 +/- 0,5% eingestellt. Ferner wurde die Luftmenge zur Oxidation so eingestellt, dass bei dem eingestellten Propenumsatz der Restsauerstoffanteil im Gasstrom nach einmaligem Durchgang durch den Reaktor 6 Vol.-% betrug. Bei allen Katalysatoren betrug die Summenausbeute für Acrolein und Acrylsäure immer mindestens 91%, wobei die Ausbeute für Acrolein immer mindestens 84% betrug. Bei allen Tests mit den Katalysatoren auf Basis der Aktivmassen der Beispiele 2a bis 2c und 3a bis 3b wurde die jeweilige maximale Temperatur im Katalysatorfestbett ermittelt.

Tabelle 2: Übersicht über die Temperaturen in den Reaktionen des Beispiels 7.

| Katalysator (aus Beispiel) | GHSV [$h^1$] | Propylen [Vol.-%] | $T_{Bad,Reaktor}$ [°C] | $T_{max,Reaktor}$ [°C] |
|---|---|---|---|---|
| 2a (erfindungsgemäß) | 2129 | 7,16 | 345 | 422 |

(fortgesetzt)

| Katalysator (aus Beispiel) | GHSV [h$^1$] | Propylen [Vol.-%] | $T_{Bad,Reaktor}$ [°C] | $T_{max,Reaktor}$ [°C] |
|---|---|---|---|---|
| 2b (erfindungsgemäß) | 2157 | 7,08 | 350 | 422 |
| 2c (erfindungsgemäß) | 2136 | 7,16 | 355 | 419 |
| 3a (Vergleichsbeispiel) | 2120 | 7,21 | > 360 | 437 |
| 3b (Vergleichsbeispiel) | 2121 | 7,19 | > 360 | 435 |

**Beispiel 8:**

Die in Beispiel 5 aus den Aktivmassen der Beispiele 3a bis 3b und 4a bis 4b hergestellten Katalysatoren wurden in der partiellen Gasphasenoxidation von Propen eingesetzt. Hierfür wurde ein Rohrreaktor mit einem Rohrrinnendurchmesser von 20,5 mm mit einem Katalysatorfestbett mit einer Länge von jeweils 285 cm beschickt. Der Rohrreaktor wurde von einem Bad umgeben, mit dem die Temperatur des Katalysatorfestbetts bzw. des das Katalysatorfestbett durchströmenden Reaktionsgemisches reguliert wurde. Ein Feedgasstrom aus Propen (ca. 7 Vol.-%, chemical grade), Luft und Inertstoffen wurde in den Rohrreaktor eingeleitet und das Propen an dem Katalysatorfestbett umgesetzt. Die zugeführte Menge an Propen wurde so gewählt, dass der Quotient aus Propengasmenge in Litern pro Stunde und der eingesetzten Füllmenge des Katalysators in Litern einen Wert von 146 h$^{-1}$ hat. Der Propenumsatz wurde durch die Wahl der Badtemperatur auf einen Wert von 97 +/- 0,5% eingestellt. Ferner wurde die Luftmenge zur Oxidation so eingestellt, dass bei dem eingestellten Propenumsatz der Restsauerstoffanteil im Gasstrom nach einmaligem Durchgang durch den Reaktor 6 Vol.-% betrug. Bei allen Katalysatoren betrug die Summenausbeute für Acrolein und Acrylsäure immer mindestens 90%, wobei die Ausbeute für Acrolein immer mindestens 84% betrug. Bei allen Tests mit den Katalysatoren auf Basis der Aktivmassen der Beispiele 2a bis 2c und 3a bis 3b wurde die jeweilige maximale Temperatur im Katalysatorfestbett ermittelt.

Tabelle 3: Übersicht über die Temperaturen in den Reaktionen des Beispiels 8.

| Katalysator (aus Beispiel) | GHSV [h$^1$] | Propylen [Vol.-%] | $T_{Bad,Reaktor}$ [°C] | $T_{max,Reaktor}$ [°C] |
|---|---|---|---|---|
| 4a (erfindungsgemäß) | 2057 | 7,09 | 354 | 423 |
| 4b (erfindungsgemäß) | 2067 | 7,06 | 357 | 425 |
| 3a (Vergleichsbeispiel) | 2036 | 7,16 | 352 | 437 |
| 3b (Vergleichsbeispiel) | 2025 | 7,21 | 360 | 435 |

**Patentansprüche**

1.  Verfahren zur Herstellung eines Mischoxidkatalysators, umfassend die Schritte

   a) Vorlegen einer Lösung und/oder Suspension von Vorläuferverbindungen von Komponenten des Mischoxidkatalysators,
   b) Sprühtrocknen der in Schritt a) vorgelegten Lösung und/oder Suspension im Gleichstrom zusammen mit einem Gasstrom, der eine Eintrittstemperatur in den Sprühtrockner von 150 +/- 10°C bis 220 +/- 10°C und eine Austrittstemperatur aus dem Sprühtrockner von 80 +/- 10°C bis 110 +/- 10°C aufweist, oder
   b') Sprühtrocknen der in Schritt a) vorgelegten Lösung und/oder Suspension im Wesentlichen im Gleichstrom zusammen mit einem Hauptgasstrom, der eine Eintrittstemperatur in den Sprühtrockner von 250 +/- 10°C bis 350 +/- 10°C und eine Austrittstemperatur aus dem Sprühtrockner von 110 +/- 10°C bis 140 +/- 10°C aufweist, wobei zwischen Eintritts- und Austrittspunkt des Hauptgasstroms in den Sprühtrockner bzw. aus dem Sprühtrockner ein zusätzlicher Gasstrom mit einer Eintrittstemperatur von weniger als 100°C in den Sprühtrockner zugeführt wird, und

c) Kalzinieren der aus Schritt b) oder b') erhaltenen Sprühpartikel unter Erhalt einer katalytischen Aktivmasse.

2. Verfahren gemäß Anspruch 1, wobei in Schritt b) der Gasstrom eine Eintrittstemperatur in den Sprühtrockner von 160 +/- 10°C bis 200 +/- 10°C in den Sprühtrockner und eine Austrittstemperatur aus dem Sprühtrockner von 90 +/- 10°C bis 105 +/- 10°C aus dem Sprühtrockner aufweist.

3. Verfahren gemäß Anspruch 1 oder 2, wobei in Schritt b) der Gasstrom eine mittlere Strömungsgeschwindigkeit im Sprühtrockner von 2,0 +/- 0,3 cm/s bis 4,5 +/- 0,3 cm/s aufweist.

4. Verfahren gemäß Anspruch 1, wobei in Schritt b') der Hauptgasstrom eine Eintrittstemperatur in den Sprühtrockner von 260 +/- 10°C bis 300 +/- 10°C und eine Austrittstemperatur aus dem Sprühtrockner von 115 +/- 10°C bis 130 +/- 10°C aufweist.

5. Verfahren gemäß Anspruch 1 oder 4, wobei in Schritt b') der zusätzliche Gasstrom mit einer Eintrittstemperatur von maximal 30°C in den Sprühtrockner zugeführt wird.

6. Verfahren gemäß einem beliebigen der Ansprüche 1, 4 oder 5, wobei in Schritt b') der Hauptgasstrom eine mittlere Strömungsgeschwindigkeit im Sprühtrockner von 2,0 +/- 0,3 cm/s bis 4,5 +/- 0,3 cm/s aufweist.

7. Verfahren gemäß einem beliebigen der Ansprüche 1 oder 4 bis 6, wobei in Schritt b') der zusätzliche Gasstrom eine mittlere Strömungsgeschwindigkeit von weniger als 2,0 cm/s aufweist.

8. Verfahren gemäß einem beliebigen der Ansprüche 1 oder 4 bis 7, wobei in Schritt b') der zusätzliche Gasstrom in einem Winkel von 90 +/- 5° bis 0° relativ zur Wand des Sprühtrockners nach oben in Richtung der Achse des Sprühtrockners zugeführt wird.

9. Verfahren gemäß einem beliebigen der Ansprüche 1 oder 4 bis 8, wobei in Schritt b') der zusätzliche Gasstrom an einem Punkt in den Sprühtrockner zugeführt wird, der sich zwischen einem Drittel oder Dreiviertel der Strecke zwischen dem Eintritts- und dem Austrittspunkt des Hauptgasstroms in den Sprühtrockner befindet.

10. Verfahren gemäß einem beliebigen der Ansprüche 1 bis 9, wobei die in Schritt a) vorgelegte Lösung und/oder Suspension Vorläuferverbindungen von sämtlichen Komponenten des Mischoxidkatalysators enthalten.

11. Verfahren gemäß einem beliebigen der Ansprüche 1 bis 9, wobei in Schritt a) Lösungen und/oder Suspensionen mit Vorläuferverbindungen von jeweils unterschiedlichen Komponenten des Mischoxidkatalysators eingesetzt und die Schritte a) und b) oder a) und b') mehrfach wiederholt werden.

12. Verfahren gemäß einem beliebigen der Ansprüche 1 bis 11, zusätzlich umfassend die Schritte

d) gegebenenfalls Vermischen der aus Schritt c) erhaltenen katalytischen Aktivmasse, sofern in Schritt b) oder b') Lösungen und/oder Suspensionen von Vorläuferverbindungen jeweils unterschiedlicher Komponenten des Mischoxidkatalysators verwendet werden, unter Erhalt einer sämtliche Komponenten des Mischoxidkatalysators enthaltenden katalytischen Aktivmasse,

e) Hinzugabe eines Form- und/oder Bindemittels zu der aus Schritt c) oder d) erhaltenen katalytischen Aktivmasse,

f) Formen der aus Schritt e) erhaltenen katalytischen Aktivmasse unter Erhalt eines den Mischoxidkatalysator enthaltenden Formkörpers, und

g) Trocknen und/oder Kalzinieren und/oder Tempern des aus Schritt f) erhaltenen Katalysatorformkörpers.

13. Verfahren gemäß einem beliebigen der Ansprüche 1 bis 11, zusätzlich umfassen die Schritte

h) Bereitstellen einer Washcoat-Suspension enthaltend die aus Schritt c) erhaltene katalytische Aktivmasse,

i) Aufbringen der Washcoat-Suspension aus Schritt h) auf ein Trägermaterial, und

j) Trocknen und/oder Kalzinieren und/oder Tempern des aus Schritt i) erhaltenen beschichteten Trägermaterials unter Erhalt eines geträgerten Mischoxidkatalysators.

**14.** Washcoat-Suspension, enthaltend eine nach einem Verfahren gemäß einem beliebigen der Ansprüche 1 bis 11 erhaltene oder erhältliche katalytische Aktivmasse.

**15.** Verwendung eines gemäß einem beliebigen der Ansprüche 1 bis 13 erhaltenen oder erhältlichen Mischoxidkatalysators in der Herstellung von ungesättigten Aldehyden und/oder Säuren durch Partialoxidation von Olefinen.

EP 3 120 926 A1

Europäisches Patentamt
European Patent Office
Office européen des brevets

# EUROPÄISCHER RECHERCHENBERICHT

Nummer der Anmeldung

EP 15 17 7519

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2014/050615 A1 (ASAHI KASEI CHEM CORP) 3. April 2014 (2014-04-03) | 1-11 | INV. B01J37/00 |
| Y | * das ganze Dokument * -& EP 2 902 105 A1 (ASAHI KASEI CHEMICALS CORP [JP]) 5. August 2015 (2015-08-05) * Absätze [0043], [0058] - [0062], [0067] * ----- | 12-15 | B01J37/08 B01J35/02 B01J23/889 B01J27/199 ADD. |
| X | JP 2006 055682 A (ASAHI KASEI CHEMICALS CORP) 2. März 2006 (2006-03-02) | 1-11 | B01J35/08 C07C45/35 |
| Y | * Absätze [0030], [0032] * ----- | 12-15 | C07C47/22 C07C51/25 |
| Y | WO 2008/046843 A1 (EVONIK DEGUSSA GMBH [DE]; FISCHER ACHIM [DE]; LU WEIMIN [CN]; WECKBECK) 24. April 2008 (2008-04-24) * Seite 4, Zeile 24 - Zeile 28 * * Seite 7, Zeile 28 - Seite 9, Zeile 1 * * Seite 9, Zeile 23 - Zeile 30 * ----- | 12-15 | B01J23/89 |
| A | WO 2014/195157 A1 (EVONIK DEGUSSA GMBH [DE]; STEFFAN MARTIN [SG]; MÜLLER HELMUT [DE]; ROT) 11. Dezember 2014 (2014-12-11) * Anspruch 8 * ----- | 15 | |

RECHERCHIERTE SACHGEBIETE (IPC)

B01J
C07C

Der vorliegende Recherchenbericht wurde für alle Patentansprüche erstellt

| Recherchenort | Abschlußdatum der Recherche | Prüfer |
|---|---|---|
| München | 17. Februar 2016 | Beckmann, Oliver |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 17 7519

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-02-2016

| Im Recherchenbericht angeführtes Patentdokument | Datum der Veröffentlichung | Mitglied(er) der Patentfamilie | Datum der Veröffentlichung |
|---|---|---|---|
| WO 2014050615 A1 | 03-04-2014 | CN 104661745 A | 27-05-2015 |
| | | EP 2902105 A1 | 05-08-2015 |
| | | KR 20150011830 A | 02-02-2015 |
| | | TW 201420184 A | 01-06-2014 |
| | | US 2015231604 A1 | 20-08-2015 |
| | | WO 2014050615 A1 | 03-04-2014 |
| EP 2902105 A1 | 05-08-2015 | CN 104661745 A | 27-05-2015 |
| | | EP 2902105 A1 | 05-08-2015 |
| | | KR 20150011830 A | 02-02-2015 |
| | | TW 201420184 A | 01-06-2014 |
| | | US 2015231604 A1 | 20-08-2015 |
| | | WO 2014050615 A1 | 03-04-2014 |
| JP 2006055682 A | 02-03-2006 | JP 5078222 B2 | 21-11-2012 |
| | | JP 2006055682 A | 02-03-2006 |
| WO 2008046843 A1 | 24-04-2008 | BR PI0718485 A2 | 03-12-2013 |
| | | CN 101164694 A | 23-04-2008 |
| | | CN 101557873 A | 14-10-2009 |
| | | EP 2073928 A1 | 01-07-2009 |
| | | JP 5550343 B2 | 16-07-2014 |
| | | JP 2010506711 A | 04-03-2010 |
| | | MY 150224 A | 31-12-2013 |
| | | RU 2009118819 A | 20-02-2011 |
| | | US 2010286450 A1 | 11-11-2010 |
| | | US 2011263415 A1 | 27-10-2011 |
| | | WO 2008046843 A1 | 24-04-2008 |
| WO 2014195157 A1 | 11-12-2014 | SG 11201509776V A | 30-12-2015 |
| | | WO 2014195157 A1 | 11-12-2014 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2155376 A1
- WO 2005049200 A1
- WO 2010028977 A1

**In der Beschreibung aufgeführte Nicht-Patentliteratur**

- **L. ZHANG et al.** *Applied Catalysis A: General,* 1994, vol. 117, 163-171